## Europäisches Patentamt

## European Patent Office

## Office européen des brevets

(19)

(11) Publication number: **0 127 400**
**A2**

(12)

# EUROPEAN PATENT APPLICATION

(21) Application number: **84303376.2**

(22) Date of filing: **17.05.84**

(51) Int. Cl.³: **A 61 K 9/20**

(30) Priority: **31.05.83 US 499646**
**09.04.84 US 596956**

(43) Date of publication of application:
**05.12.84 Bulletin 84/49**

(84) Designated Contracting States:
**BE CH DE FR GB LI NL**

(71) Applicant: **STAUFFER CHEMICAL COMPANY**

**Westport Connecticut 06881(US)**

(72) Inventor: **Pfann, John Richard**
**165 Stella Lane**
**Fairfield Connecticut 06430(US)**

(74) Representative: **Smith, Sydney et al,**
**Elkington and Fife High Holborn House 52/54 High Holborn**
**London WC1V 6SH(GB)**

(54) **Magnesium oxide containing vehicle for direct compression tableting.**

(57) Direct compression magnesium containing vehicle, preferably magnesium oxide, is prepared by admixing a magnesium compound with a calcium phosphate, compacting under sufficient pressure to form a sheet and comminuting the granules to a particle size sufficient for the desired end use such as direct compression tableting. The cocompacted blend can be used as is or as the base for dilution with additional ingredients.

Croydon Printing Company Ltd

EP 0 127 400 A2

-1-

# MAGNESIUM OXIDE CONTAINING VEHICLE FOR DIRECT COMPRESSION TABLETING

## BACKGROUND OF THE INVENTION

There are two general methods for forming tablets, i.e. compression of a dry particulate material or molding of a moist material. In compressing a dry particulate material, dry direct compression, wet granulation or dry granulation techniques can be used. The direct compression technique is the most desirable in that it employs the fewest steps and, in the case of production of tablets containing sensitive or unstable active materials, minimizes the exposure to water or other conditions tending to adversely affect the stability of the active ingredient. However, the application of the direct compression technique is limited.

Since most active materials possess poor compression properties or are used in such small amounts per unit dose that direct compression is impractical, the active ingredient must be admixed with a direct compression vehicle, i.e. a composition which is compatible with the active ingredient and has good compressibility. In addition, the direct compression vehicle must have good flowability, good stability under normal ambient conditions, no adverse effect on tablet disintegration time, the ability to produce good tablet surfaces and low cost. Popular compression vehicles such as spray dried lactose possess poor stability with

CIP of C-6954

certain active ingredients and discolor on storing. Microcrystalline cellulose is expensive and thus has limited use.

In addition to the inclusion of the active ingredient and the direct compression vehicle in the tablet, a tableting formulation normally includes other additives such as diluents, lubricants, flavors, colors, disintegration agents and the like. When the tableting formulation includes a large number of components, direct compression techniques can be even less useful because of the difficulty of assuring uniform mixing of the various components on dry blending.

In addition, direct compression tableting is usually accomplished using a high-speed rotary tableting machine. The feeding apparatus for these machines is generally a gravity type feed which can be seriously affected by caking or bridging. Improper feed can result in non-uniformity of tablet weight. Any tableting formulation must be sufficiently flowable under the conditions of tableting to provide good tablets.

In the field of vitamin and mineral supplements manufacturers would like to provide, as a source of magnesium, a direct compression vehicle containing magnesium oxide. Magnesium oxide powder is unacceptable because of the caking and bridging problems that it causes in a direct-compression rotary tableting machine. Granular magnesium oxide is sold commercially and is used in direct compression tableting to insure that the flow from the hopper into the rotary press is not affected. One manner of preparing granular magnesium oxide requires the compacting of a magnesium oxide in a roller compactor such as a Chilsonator ® sold by Fitzpatrick. However, to avoid destruction of the roller compactor, it has been found necessary to add a lubricant to the magnesium oxide. The lubricant which is used is that which is generally compatible with the tableting system. The most preferred lubricant is magnesium stearate. Other magnesium oxide granular products are available which do not contain magnesium stearate.

However, the tableting manufacturer generally adds a lubricant such as magnesium stearate in the final tablet for his tableting machine. Since the level of magnesium stearate in the final tablet is usually a constant amount based on the weight of the final tablet, the presence of the magnesium stearate in the magnesium oxide vis-a-vis a magnesium oxide granular without magnesium stearate could cause potential formulation problems.

Further, the vitamin manufacturers would also like to use the direct compression vehicle as a source of calcium and phosphorus.

## SUMMARY OF THE INVENTION

In accordance with the present invention, it has been found that a direct compression granular material containing a magnesium composition that

CIP of C-6954

can provide calcium and phosphorus can be prepared by dry blending a magnesium compound in the oxide, hydroxide, carbonate or phosphate salt form with a calcium phosphate followed by cocompacting the blend under mechanical pressure of from about 300 to about 1000 atmospheres. The process can be carried out statically in an hydraulic press or dynamically between a pair of counterrotating rolls. Lubricants are not required for compaction. The resulting pellets, sheets or ribbons of compacted material are then broken into smaller size particles and screened to separate the granules having a particle size useful for direct compression. These granules are useful as direct compression vehicles in producing tablets by direct compression.

## DETAILED DESCRIPTION OF THE INVENTION

The magnesium salt for use in the invention is preferably the magnesium oxide though the hydroxide, carbonate or phosphate (mono, di or tri) can also be used. The magnesium compound must be in the form of a fine powder having a particle size ranging from about 0.5 to about 75 microns.

It has also been found that magnesium oxide materials prepared by various methods can provide differing results. While effective results have been achieved using various magnesium oxides, enhanced compressibility profiles (increase in tablet hardness with corresponding increases in tablet

CIP of C-6954

compaction force until terminated by capping, the point at which the compressibility of the material is exceeded with the resultant breaking off of the top and bottom of the tablets) have been shown using a magnesium oxide of less than 44 micron particle size (through 325 mesh) with a loose bulk density ranging from about 128 to about 240 kilograms per cubic meter. A particularly preferred material is made by a process that broadly includes the steps of a) slaking and carbonating with carbon dioxide a dolomitic limestone; b) decarbonating under pressure the recovered magnesium bicarbonate from step a); and c) calcining the recovered magnesium carbonate from step b) at a temperature between about 700°C and 1100°C to recover the desired magnesium oxide. This product is available having a particle size of less than 44 microns and a bulk density of from about 128 to 240 kilograms per cubic meter. It has been unexpectedly found that this material provides a wider compressibility range before capping allowing for the formation of harder tablets. This product is available from Martin Marietta Chemicals under the trademark MagChem food grade/ USP grade (as of this writing, also identified by Martin Marietta as "heavy").

An alternate process for preparing magnesium oxide from dolomite is shown in U.S. 3,402,017.

The calcium phosphates for use in the invention can include monocalcium, monocalcium monohydrate, anhydrous dicalcium, dihydrated dicalcium, anhydrous tricalcium or hydroxyapatite, calcium pyrophosphate and mixtures thereof. Preferably, the calcium phosphate is the di- or tricalcium phosphate and more preferably the tricalcium phosphate. For effective compaction, calcium phosphate having a particle size generally ranging from about 0.5 to about 75 microns is used.

The magnesium compound and the calcium phosphate, preferably magnesium oxide and tricalcium phosphate, with particle sizes ranging from 0.5 to 75 microns are blended using any industrially available blending equipment. Preferably, the blending is accomplished under low shear such as in a V-blender or ribbon blender. Any suitable industrial equipment that allows for adequate mixing of the two materials without destruction can be used.

The magnesium compound and the calcium compound can be cocompacted in amounts sufficient to provide Ca:Mg ratios ranging from about 10:1 to about 1:20. This broad ratio range is satisfied with a ratio of tricalcium phosphate to magnesium oxide ranging from about 95%/5% to about 5%/95%. Two preferred ratio ranges have been identified, i.e. from about 0.5:1 to about 5:1 and more preferably from about 1:1 to about 3:1 as well as from about 0.3:1 to about 0.05:1 and preferably from about 0.2:1 to about 0.075:1.

The first preferred ratio range can be satisfied by a ratio of tricalcium phosphate to magnesium oxide of from about 88%/12% to 43%/57% and more preferably 82%/18% to 60%/40%. The most preferred ratio within the first preferred ratio is 70%/30% which provides the same calcium to magnesium ratio as in dolomite

CIP of C-6954

(calcium magnesium carbonate) which is frequently used as a source of magnesium in vitamin tablets.

The second preferred ratio range can be satisfied by a ratio of tricalcium phosphate to magnesium oxide of from about 31%/69% to about 7%/93% and more preferably from about 23%/77% to about 10%/90%. Two preferred embodiments within that range are about 20%/80% and about 10%/90% though other ratios such as 15%/85% are effective.

The conversion of the fine particles of the blend to granules useful as a direct compression vehicle is carried out in two steps: compaction and comminution.

The blend can be compacted by the static application of pressure as in an hydraulic press. Preferably, the blend is compressed by feeding the blend into the nip of the counterrotating rolls of a roller press. The rolls can counterrotate at an rpm ranging from about 7 to about 100 rpm generating pressures of up to 3000 atmospheres. The blend is fed to the rolls using a feed system which forms a densified powder mass behind the nip. The powder is densified sufficiently for controlled volume flow. The densified powder mass is forced between the two rolls of a roller compactor for controlled compaction. The roller compactor can be of the type sold under the trademark CHILSONATOR as described in U.S. Patent 3,255,285.

The compaction step can be carried out at a high or low rate of application of pressure. The rate of application of pressure in a rolling press can be as high as about 10,000 atmospheres per second

CIP of C-6954

whereas in an hydraulic press the rate can be 50-100 atmospheres per second. Irrespective of the rate of application of pressure, which is determined by the type of apparatus employed, the actual pressure employed for the compaction step can vary from about 200 atmospheres to about 3000 atmospheres or higher. The preferred range in applied pressure for compaction is from about 300 atmospheres to about 1000 atmospheres. For a set of counterrotating rolls the preferred range of linear hydraulic force is from about 700 to about 30,000 kg/cm; the more preferred range is from 1000 to about 25,000 kg/cm.

The size of the sheet or ribbon produced by compaction is determined by the apparatus employed. A laboratory hydraulic press can make a sheet about 10 cm to 40 cm square. Commercial rolling presses are available to produce a ribbon from about 6 cm to about 60 cm wide and about 25 mm to about 40 mm thick. The thickness in the case of the hydraulic press is determined by the amount of powder charged. In the case of the rolling press, the thickness of the ribbon produced is determined by the rate of feed of powder into the nip between the rolls, as well as the configuration of the rolls and the pressure applied to the rolls.

The particle size of the fine powder used as the feed material varies from about 0.5 micron to about 75 microns. The bulk density of the fine particle feed to the compacting process of the present invention ranges from about 0.15 to about 0.5 g/cc (10-30 lbs/ft$^3$).

CIP of C-6954

The surface of the rolls or plates applying the mechanical force can be scored to give corrugated, patterned, or briquetted compacted product of any desired shape. Corrugations facilitate the flow of the fine feed into the nip of the rolls.

When rolls are used to compact the blend in ribbon form, they can rotate at any speed from about 1 to about 100 rpm. Within this range a speed of from about 7 to about 20 rpm is preferred.

The invention can be carried out at low relative humidity and ambient temperature. The compaction **step in exerting mechanical** pressure on the fine particles of the calcium phosphate/magnesium compound blend by means of rolls increases the temperature from about 10°C to about 30°C. Chilling the pressure rolls is unnecessary unless discoloration of the compacted material commences.

Preferably, the comminution step is carried out in two steps: prebreaking and grinding. The compacted ribbon or sheet of the blend can be broken up into flakes, chips, slices, or pieces by standard cutting machines if necessary. It is convenient to attach a rotating set of cutting knives just below the compacting chamber of a continuous rolling press so that the ribbon of compacted material is immediately broken up into pieces varying in size from about 5 millimeters to 50 millimeters. The speed of rotation of the prebreaking knives varies from about 40 to about 1000 rpm, thus determining the size of the pieces.

To be useful as a direct compression vehicle the compacted blend is ground to a size of from about 80 microns to about 400 microns having a bulk density of from about 0.7 to about 1.1 g/cc.

Grinding mills such as those manufactured by the Fitzpatrick Company, Elmhurst, Illinois; Pulverizing Machinery Company, Summit, New Jersey; and Raymond Division of Combustion Engineering Company, Stamford, Connecticut, can be used to prepare the desired sized granules from the compacted sheet, ribbon, flakes or chips. The particle size of the comminuted product is determined by a number of parameters in a grinding mill among which are the number of cutting blades, the shape of the cutting blades, the speed of rotation of the blades, the size of the screen employed, the shape of the holes in the screen, the type of feed throat, the pattern at which the blades are set, and the rate at which the feed is fed into the comminutor.

After comminuting, the product is classified to provide the desirable particle size for a direct compression vehicle. Granules having a particle size ranging from about 80 to about 400 micron are preferred. Any appropriate screening or sieving device can be used including screens, perforated plates, cloth and the like. Vibratory devices can be attached to assist in the particle separation. Air separators and the like can be used if appropriate classification of particles can be obtained.

CIP of C-6954

Following classification, the particles having the desired particle size range are segregated for use as a direct compression vehicle. The fine and large particles are recycled to the compactor for further processing.

While it is generally preferred to prepare the direct compression vehicle without further additives, additives can be added to the blend prior to compaction for improvement of specific properties. For example, binders such as starches, ethyl cellulose, and gums may be added. Lubricants, disintegrants can be added though these are usually added, if required, during blending prior to tableting. Compression improvers such as microcystalline cellulose can also be added in amounts effective to achieve desired tablet properties. Preferably, further ingredients are not added prior to compaction to allow the tablet manufacturer more latitude in tableting since no special benefit is obtained by early addition.

The products of the invention can be made into tablets or dry capsules by direct compression using well known techniques. Prior to tableting, the active ingredient along with other ingredients such as binders (starches), disintegrants, color, flavor, diluents and any other materials normally used in tablets can be blended with the granules. A lubricant is usually blended last to coat the surface of the particles and provide exterior lubrication.

CIP of C-6954

After mixing, the blend can be formed into a tablet by dry, direct compression. Either single hydraulic units or multiple anvil high-speed rotary tableting units can be used as known in the industry. The tablets can be formed in the shape of round tablets or dry capsules, as desired, with equivalent results. The composition of the present invention can be compressed at a high rate of compression in a rotary tableting machine as well as a low rate of compression utilizing a single tablet hydraulic unit with effective results.

The hardness and compressibility can be improved by the addition of up to about 25% microcrystalline cellulose. Surprisingly, it has been found that a marked improvement can be obtained by using a microcrystalline cellulose in combination with a product of the invention made with the material previously identified as MagChem.

The invention is illustrated in the Examples which follow. All percentages and ratios are by weight unless otherwise stated.

EXAMPLE 1

270 kg (600 lbs.) of USP grade magnesium oxide (a.k.a. "heavy"), manufactured by Martin Marietta Company, and 628 kg (1395 lbs.) of NF/FCC grade of anhydrous tricalcium phosphate, manufactured by Stauffer Chemical Company were charged into a ribbon blender and blended for 1.5 hours. An additional 50 kg (110 lbs.) of tricalcium phosphate was then added to the mixture which remained in the blender.

The mixture thus prepared was compacted in a Fitzpatrick 4X10 Chilsonator system. This Chilsonator was provided with rolls of 10 cm (4 inches) in width and 25 cm (10 inches) in diameter. The rolls had a sine-wave surface and were separated with a roll gap of 0.05 cm (0.020 inches). The powder mixture was fed to the Chilsonator by a vertical-screw type conveyor into the Chilsonator and compacted by the rolls. One roll was hydraulically forced against the other with a pressure of 64 kg/sq cm gauge (910 psig). The rolls had a rotational speed of 18 rpm.

The compacted product was in the form of broken corrugated sheets. The compacted product was directly charged to a Fitzmill (Model DAS06) equipped with rotating knife-blades. The product was discharged from the mill through a screen which had round openings of 0.125 cm (0.05 inch).

The compacted and milled product was directly fed onto a vibrating screening unit. The screens

CIP of C-6954

used were 120 cm (48 inches) in diameter.  The first screen was rated at 36 TBC (tensile bolting cloth). The second screen was rated at 78 TBC.  These vibrating screens separated the feed into three portions.  The product cut was taken from the middle portion, i.e., particles that passed the 36 TBC screen but could not pass the 78 TBC screen.  The oversize and undersize fractions that came out of the vibrating screens were discharged into the Chilsonator feed hopper, blended with the raw feed for the Chilsonator and recycled.

A sieving analysis performed on the product showed there were no particles coarser than 20 mesh and that a majority of the product had particle sizes between 20 and 60 mesh (U.S. Standard Sieve Series).

EXAMPLE 2

182 kg (400 lbs.) of MARINCO®OH grade of magnesiu oxide, manufactured by Merck and Company, and a total of 422 kg (928 lbs.) of the NF/FCC grade of anhydrous tricalcium phosphate, manufactured by Stauffer Chemical Company, were charged into a ribbon blender, blended for 1.5 hours, and discharged.

60 kg (1011 lbs.) of this mixture was then recharged to the blender.

Then, an additional 44 kg (97.5 lbs.) of tricalcium phosphate were added to the blender.

After an additional 1.5 hours of blending, the blender was randomly sampled.

The powder was compacted in a Fitzpatrick 4X10 Chilsonator system as described in Example 1.

The compacted product was in the form of broken corrugated sheets. The compacted product was directly charged to a Fitzmill (Model DAS06) equipped with rotating knife-blades. The product was discharged directly from the mill through a screen which had round openings of 0.125 cm (0.05 inch).

The compacted and milled product was screened as in Example 1. The product cut was taken from the middle portion, i.e., particles that passed the 36 TBC screen but could not pass the 78 TBC screen. The over- and undersize fractions were blended with the raw feed for the Chilsonator and recycled.

A sieving analysis performed on the product showed there was no particle coarser than 20 mesh and a majority of the particles had a particle size between 20 and 60 mesh (U.S. Standard Sieve Series).

CIP of C-6954

TABLET PREPARATION

Tablets were prepared using the formulations set forth below by placing all ingredients except the magnesium stearate lubricant in a twin shell V-blender (Patterson-Kelly) equipped with an intensifier bar. After mixing for 5 minutes with the intensifier bar on, 0.5% magnesium stearate was added and the mixture was blended for an additional 2 minutes with the intensifier bar off.

The formulations were formed into tablets by dry direct compression on a rotary tableting machine (Manesty B3B) equipped with 7/16 inch IPT standard cup tooling. The tableting machine was equipped with strain gauges attached to a recorder to record the compression force applied to each tablet run. Four stations out of a possible 16 tableting stations on the machine were used. Tablets were produced at a rate of 750 tablets per minute based on 16 stations. Nominal tablet weight was 750 milligrams.

The tablets were tested for hardness using a Schleuniger Tablet Hardness Tester, Model 2E/106. Hardness is reported as an average of the hardness of 10 tablets.

The percentages are weight percent. All tablet formulations contain 0.5% lubricant. The lubricant was magnesium stearate. The following results were obtained:

| | Ingredients | Compression Force (Kilonewtons) | Tablet Hardness (Kiloponds) |
|---|---|---|---|
| A) | Control | | |
| | 99.5% TCP | | |
| | Tablet Run | | |
| | 1 | 8.1 | 4.0 |
| | 2 | 11.6 | 5.9 |
| | 3 | 16.2 | 8.0 |
| | 4 | 19.9 | 9.2 |
| | 5 | 23.6 | 11.6 |
| B) | Control | | |
| | 69.5% TCP/30% MgO Dry Blend | | |
| | Tablet Run | | |
| | 1 | 7.7 | 2.8 |
| | 2 | 9.9 | 3.6 |
| | 3 | 11.8 | 4.3 |
| C) | 99.5% Product Example 2 (Cocompacted MgO/TCP) | | |
| | Tablet Run | | |
| | 1 | 8.1 | 3.2 |
| | 2 | 10.6 | 3.6 |
| | 3 | 11.8 | 4.7 |
| | 4 | 14.0 | 5.8 |
| | 5 | 16.1 | 6.2 |

CIP of C-6954

| Ingredients | Compression Force (Kilonewtons) | Tablet Hardness (Kiloponds) |
|---|---|---|
| D) 99.5% Product Example 1 (Cocompacted MgO/TCP) | | |
| Tablet Run | | |
| 1 | 8.1 | 4.1 |
| 2 | 10.5 | 5.0 |
| 3 | 15.8 | 7.5 |
| 4 | 19.8 | 9.0 |
| 5 | 23.6 | 10.8 |
| 6 | 27.2 | 12.6 |
| E) 84.5% Product Example 2/ 15% Microcrystalline Cellulose | | |
| Tablet Run | | |
| 1 | 7.8 | 0.0 |
| 2 | 11.8 | 6.5 |
| 3 | 15.9 | 9.0 |
| 4 | 20.2 | 10.4 |
| 5 | 23.3 | 11.2 |
| 6 | 27.9 | 13.8 |
| F) 84.5% Product Example 1/ 15% Microcrystalline Cellulose | | |
| Tablet Run | | |
| 1 | 8.3 | 5.1 |
| 2 | 10.3 | 6.3 |
| 3 | 16.5 | 10.0 |
| 4 | 19.9 | 12.9 |
| 5 | 24.0 | 15.4 |
| 6 | 28.9 | 17.8 |

CIP of C-6954

| Ingredients | Compression Force (Kilonewtons) | Tablet Hardness (Kiloponds) |
|---|---|---|
| G) 69.5% TCP/30% MgO/ 15% Avicel (Coblended) | | |
| Tablet Run | | |
| 1 | 7.9 | 4.4 |
| 2 | 11.5 | 5.9 |
| 3 | 15.5 | 7.9 |
| 4 | 20.6 | 9.4 |
| 5 | 22.8 | 10.1 |
| 6 | 24.5 | 10.9 |

The TCP used in the above Examples A, B and G is direct compression granular tricalcium phosphate sold under the trademark TRI-TAB by Stauffer Chemical Company. The microcrystalline cellulose is sold under the trademark AVICEL PH 101. The magnesium oxide used in Examples B and G is a directly compressible granular product.

Compressibility profiles of these tablets can be prepared by graphing the above data with the compression force on the X axis and the hardness on the Y axis. The compressibility profile of a dry blend of directly compressible tricalcium phosphate/ magnesium oxide (Example B) is significantly below that of directly compressible tricalcium phosphate alone (Example A). The cocompacted blend of Example 2 is better than the dry blend but still lacks in hardness and compressibility. The addition of 15%

CIP of C-6954

microcrystalline cellulose increases the profile to that of the direct compression tricalcium phosphate. The cocompacted product of Example 1 is approximately equivalent to the direct compression tricalcium phosphate and also can be compacted at higher force to provide additional hardness. The addition of microcrystalline cellulose to the product of Example 1 provides increased hardness for a limited compaction force.

EXAMPLE 3

A 80% MgO/20% TCP cocompacted product was prepared by blending for 3 hours 727 kilograms of heavy food grade magnesium oxide, manufactured by Martin Marietta Company, and 182 kilograms of NF/FCC grade anhydrous tricalcium phosphate, manufactured by Stauffer Chemical Company, in a ribbon blender and discharged.

The mixture was then compacted by feeding the powder mixture to a Fitzpatrick Chilsonator$^{TM}$ compactor system. This Chilsonator was provided with rolls of approximately 30 centimeters in width and 30 centimeters in diameter. The rolls had a sine-wave surface. One roll was hydraulically forced against the other with a pressure of 120 kilograms/square centimeter gauge.

The compacted product in the form of broken corrugated sheets was directly charged to a Fitzmill equipped with knife-blades rotating at 3000 revolutions per minute. The product was discharged from the mill through a screen which had round openings of 0.20 centimeter.

The compacted and milled product was directly fed onto a vibrating screening unit manufactured by the Kason Corporation. The screens used were 183 centimeters in diameter. The first screen was rated at 36 Tensile Bolting Cloth. The second screen was rated at 76 Tensile Bolting Cloth. These vibrating screens separated the feed into three portions.

CIP of C-6954

The product cut was taken from the middle portion, i.e., particles that passed the 36 Tensile Bolting Cloth screen but could not pass the 76 Tensile Bolting Cloth screen. The oversize and undersize fractions that came out of the vibrating screens were discharged into the Chilsonator feed hopper, blended with the raw feed for the Chilsonator and recycled.

A sieving analysis performed on this 80% MgO/20% TCP cocompacted product showed there were no particles coarser than 20 mesh and that a majority of the product had particle sizes between 40 and 100 mesh (U.S. Standard Sieve Series).

Tablets were prepared using the formulations set forth below and tested in the same manner as in Example 2. All formulations contained 0.5% magnesium stearate as lubricant and 1.0% as a disintegrant (Ac-Di-Sol®) in addition to the magnesium oxide and tricalcium phosphate. The compacted tricalcium phosphate used in the examples is TRI-TAB® from Stauffer Chemical Company. The magnesium oxide is MagChem 500 G30 from Martin Marietta Company. The following results were obtained. (The ratios given are relative to the ratio of each specified component in the blend.)

CIP of C-6954

|  | Ingredients | Compression Force (Kilonewtons) | Tablet Hardness (Kiloponds) |
|---|---|---|---|

**80% MgO/20% TCP BLENDS**

A) Cocompacted 80% MgO/20% TCP

Tablet Run

| | | Compression | Hardness |
|---|---|---|---|
| 1 | | 10.4 | 4.6 |
| 2 | | 11.9 | 5.3 |
| 3 | | 13.6 | 6.1 |
| 4 | | 16.6 | 7.3 |
| 5 | | 17.0 | 7.9 |
| 6 | | 19.4 | 9.1 |

B) Dry Blend 19.8% Compacted TCP/80.2% (granular)

Tablet Run

| 1 | | 9.5 | 4.2 |
|---|---|---|---|
| 2 | | 10.5 | 5.1 |
| 3 | | 12.8 | 6.0 |
| 4 | | 14.2 | 6.1 |
| 5 | | 17.4 | 8.0 |
| 6 | | 19.5 | 7.8 |

C) Dry Blend of 71.1% MgO and 28.9% Cocompacted 70% TCP/30% MgO

Tablet Run

| 1 | | 10.1 | 4.0 |
|---|---|---|---|
| 2 | | 12.2 | 5.9 |
| 3 | | 14.0 | 5.8 |
| 4 | | 16.1 | 7.7 |
| 5 | | 17.3 | 8.2 |
| 6 | | 20.5 | 8.8 |

CIP of C-6954

| Ingredients | Compression Force (Kilonewtons) | Tablet Hardness (Kiloponds) |
|---|---|---|
| D) Dry Blend of 61.9% Compacted TCP and 38.1% Cocompacted 80% MgO/20% TCP (final tablet 30% MgO/ 70% TCP) | | |
| Tablet Run | | |
| 1 | 9.9 | 4.9 |
| 2 | 11.4 | 5.5 |
| 3 | 13.9 | 7.0 |
| 4 | 15.9 | 7.5 |
| 5 | 18.5 | 8.9 |
| 6 | 20.5 | 9.0 |
| E) Control Compacted TCP | | |
| Tablet Run | | |
| 1 | 10.2 | 5.5 |
| 2 | 11.5 | 6.3 |
| 3 | 14.3 | 8.3 |
| 4 | 16.0 | 9.9 |
| 5 | 18.1 | 9.8 |
| 6 | 20.6 | 12.0 |

CIP of C-6854

EXAMPLE 4

A 90% MgO/10% TCP cocompacted product was prepared from 818 kilograms of heavy food grade magnesium oxide, manufactured by Martin Marietta Company, and 91 kilograms of NF/FCC grade anhydrous tricalcium phosphate, manufactured by Stauffer Chemical Company, using the procedure of Example 3.

A sieving analysis performed on this 90% Mgo/10% TCP cocompacted product showed there were no particles coarser than 20 mesh and that a majority of the product had particle sizes between 40 and 100 mesh (U.S. Standard Sieve Series).

Tablets were prepared using the formulations set forth below and tested in the same manner as in Example 2. All formulations contained 0.5% magnesium stearate as lubricant and 1.0% as a disintegrant (Ac-Di-Sol ®) in addition to the magnesium oxide and tricalcium phosphate. The compacted tricalcium phosphate used in the examples is TRI-TAB ® from Stauffer Chemical Company. The magnesium oxide is MagChem from Martin Marietta Company. The following results were obtained. (The ratios given are relative to the ratio of each specified component in the blend.)

CIP of C-6954

| Ingredients | Compression Force (Kilonewtons) | Tablet Hardness (Kiloponds) |
|---|---|---|

### 90% MgO/10% TCP BLENDS

A) Cocompacted
   90% MgO/10% TCP

Tablet Run

| | Compression Force (Kilonewtons) | Tablet Hardness (Kiloponds) |
|---|---|---|
| 1 | 10.1 | 4.3 |
| 2 | 11.5 | 5.7 |
| 3 | 13.9 | 6.2 |
| 4 | 16.6 | 7.4 |
| 5 | 18.0 | 7.3 |
| 6 | 20.6 | 8.6 |

B) Dry Blend of
   90.3% MgO granular and
   9.7% Compacted TCP

Tablet Run

| | Compression Force (Kilonewtons) | Tablet Hardness (Kiloponds) |
|---|---|---|
| 1 | 9.6 | 4.3 |
| 2 | 11.8 | 5.5 |
| 3 | 14.0 | 6.8 |
| 4 | 16.1 | 7.1 |
| 5 | 17.9 | 8.4 |
| 6 | 20.1 | 8.3 |

C) Dry Blend of 14.2%
   Cocompacted 70% TCP/
   30% MgO and 85.8% MgO

Tablet Run

| | Compression Force (Kilonewtons) | Tablet Hardness (Kiloponds) |
|---|---|---|
| 1 | 9.5 | 3.5 |
| 2 | 12.2 | 5.3 |
| 3 | 13.7 | 5.2 |
| 4 | 15.9 | 6.5 |
| 5 | 17.2 | 6.6 |
| 6 | 20.9 | 7.2 |

| Ingredients | Compression Force (Kilonewtons) | Tablet Hardness (Kiloponds) |
|---|---|---|
| **D) Dry Blend of 66.3% Compacted TCP and 33.7% Cocompacted 90% MgO/10% TCP** | | |
| Tablet Run | | |
| 1 | 9.9 | 4.6 |
| 2 | 12.0 | 5.7 |
| 3 | 13.8 | 5.8 |
| 4 | 15.5 | 6.9 |
| 5 | 17.8 | 8.1 |
| **E) Control Compacted TCP** | | |
| Tablet Run | | |
| 1 | 10.2 | 5.5 |
| 2 | 11.5 | 6.3 |
| 3 | 14.3 | 8.3 |
| 4 | 16.0 | 9.9 |
| 5 | 18.1 | 9.8 |
| 6 | 20.6 | 12.0 |

Compressibility profiles of these tablets were prepared.

Sample D in Examples 3 and 4 was prepared as part of a different group of experiments from A, B, C and E. Since the control results in that experiment are comparable to Sample E, Sample D is considered representative.

CIP of C-6954

For comparative purposes, 30% MgO/70% TCP blends were also prepared and tested using the procedure of Example 3.

| Ingredients | Compression Force (Kilonewtons) | Tablet Hardness (Kiloponds) |
|---|---|---|
| **30% MgO/70% TCP BLENDS** | | |
| A) Dry Blend of 30% Uncompacted MgO/70% Compacted TCP | No Tablets Could Be Formed | |
| B) 30% MgO/70% TCP Cocompacted | | |
| Tablet Run | | |
| 1 | 10.1 | 5.1 |
| 2 | 11.8 | 5.8 |
| 3 | 13.9 | 7.1 |
| 4 | 16.2 | 8.1 |
| 5 | 18.0 | 9.0 |
| 6 | 20.4 | 10.6 |
| C) Control Compacted TCP | | |
| Tablet Run | | |
| 1 | 9.6 | 5.4 |
| 2 | 12.0 | 7.1 |
| 3 | 14.1 | 8.1 |
| 4 | 16.1 | 10.3 |
| 5 | 17.9 | 10.5 |
| 6 | 19.8 | 11.1 |

As it can be seen from the data reported in Examples 3 and 4, direct compression tablets having properties equivalent to tablets prepared using the control roller compacted tricalcium phosphate can be prepared by using cocompacted blends of magnesium oxide and tricalcium phosphate, i.e. 80%/20% or 90%/10%. It has also been found that tablets can be prepared using direct compression which have results equivalent to that of the tricalcium phosphate control using a dry blend of granulated magnesium oxide and roller compacted tricalcium phosphate corresponding to the same ratio. It has also been found, surprisingly, that a cocompacted blend of tricalcium phosphate and magnesium oxide can be blended with either tricalcium phosphate or magnesium oxide in order to prepare a material which is effective in direct compression tableting. This is in contrast to the fact that pure blends of tricalcium phosphate and magnesium oxide at the same final ratio are not effective in direct compression tableting.

To further assist in understanding this aspect of the invention, it is pointed out that it is known that tricalcium phosphate does not flow into a direct compression tableting die. Roller compacted tricalcium phosphate can be effectively used for direct compression tableting. Further, a blend of 70% roller compacted tricalcium phosphate and 30% powdered magnesium oxide does not flow into the dies for direct compression tableting. A blend of 70% roller compacted tricalcium phosphate and 30% granular

CIP of C-6954

(assumedly roller compacted) magnesium oxide does flow into the direct compression dies but the tablets cap (upper surface of the tablet breaks off) under even the lowest tableting pressure. One product that is effective in forming tablets by direct compression is the cocompacted blend of 70% tricalcium phosphate and 30% magnesium oxide.

Blends of 80% or 90% magnesium oxide and 20% or 10% respectively tricalcium phosphate, whether admixtured or cocompacted, can be effectively tableted by direct compression. In contrast, blends of these materials containing a high proportion of tricalcium phosphate cannot be used as a direct compression tableting material without cocompaction. In addition, cocompacted blends of tricalcium phosphate and magnesium oxide, wherein the magnesium oxide is present in an amount sufficient to allow the preparation of blends which can be tableted even though diluted, can be combined with tricalcium phosphate or magnesium oxide as diluents to prepare effective direct compression tableting material. Magnesium oxide is generally used in a majority amount in these blends.

CIP of C-6954

CLAIMS:

1. A process for preparing a directly compressible magnesium compound which comprises:

a) blending a magnesium compound of the oxide, hydroxide, carbonate or phosphate type with a calcium phosphate;

b) compacting the blend of step a) under sufficient pressure to form a sheet; and

c) comminuting said sheet to granules suitable for direct compression tableting.

2. The process as recited in Claim 1 wherein said magnesium compound is selected from the group consisting of the oxide and the hydroxide.

3. The process as recited in Claim 1 wherein said magnesium compound is magnesium oxide.

4. The process as recited in Claim 3 wherein said magnesium oxide has a particular size of less than 44 microns and a bulk density ranging from about 128 to about 240 kilograms per cubic meter.

5. The process as recited in Claim 1 wherein said calcium phosphate has a particle size ranging from about 0.5 to about 75 microns.

6. The process as recited in Claim 1 wherein said calcium phosphate is selected from the group consisting of monocalcium phosphate, dicalcium phosphate, tricalcium phosphate and calcium pyrophosphate.

CIP of C-6954

7. The process as recited in Claim 1 wherein said calcium phosphate is selected from the group consisting of dicalcium phosphate and tricalcium phosphate.

8. The process as recited in Claim 1 wherein said calcium phosphate is tricalcium phosphate.

9. The process as recited in Claim 1 wherein the compacting pressure ranges from about 300 to about 1000 atmospheres.

10. The process as recited in Claim 1 wherein said compaction is accomplished by feeding said blend to the counterrotating rolls of a rotary compactor.

11. The process as recited in Claim 1 wherein said granules have a particle size ranging from about 80 to about 400 microns.

12. A cocompacted blend of a magnesium compound of the oxide, hydroxide, carbonate or phosphate type and a calcium phosphate.

13. The cocompacted blend as recited in Claim 12 wherein said cocompacted blend is comminuted to form granules suitable for direct compression tableting.

14. A direct compression vehicle containing magnesium, calcium and phosphorus comprising the granular form of a cocompacted blend of a magnesium salt compound of the oxide, hydroxide, carbonate or phosphate type and a calcium phosphate, the granules having a particle size ranging from about 80 to about 400 microns.

CIP of C-6954

15. The process as recited in Claim 1 wherein said magnesium compound is used in an amount relative to the calcium compound sufficient to provide a calcium/magnesium ratio ranging from about 10:1 to about 1:20.

16. The product of the process of Claim 1.

17. A process of preparing tablets by direct compression which comprises dry mixing an active ingredient with the product of Claim 14 and forming a tablet by direct compression.

18. A direct compression vehicle for forming a tablet by direct compression comprising a blend of the cocompacted product of Claim 14 and an inert diluent.

19. The vehicle as recited in Claim 18 wherein said inert diluent comprises at least one of the ingredients used in preparing said cocompacted product.

20. The vehicle as recited in Claim 19 wherein the ratio of calcium to magnesium ranges from about 10:1 to about 1:20.

21. A direct compression tableting vehicle comprising a blend of a magnesium salt compound of the oxide, hydroxide, carbonate or phosphate type and a calcium phosphate, the granules having a particle size ranging from about 80 to about 400 microns wherein the ratio of calcium to phosphorus ranges from about 0.3:1 to about 0.05:1.

CIP of C-6954